# EUROPEAN PATENT APPLICATION

(11) **EP 4 413 911 A1**
(43) Date of publication of application: **14.08.2024**
(21) Application number: 23193273.2
(22) Date of filing: 24.08.2023
(51) Int. Cl.: A61B 1/00, A61B 1/06, A61B 5/00, A61B 90/00, G01N 21/64, G06V 10/82, G06V 40/16, A61B 1/04, G06T 7/00, G06T 11/00

(54) **METHOD AND SYSTEM FOR PROCESSING FLUORESCENT IMAGE GUIDED SURGICAL OR DIAGNOSTICS IMAGERY**

(30) Priority: 09.02.2023 US 202363444288 P
(71) Applicant: Olympus Winter & Ibe GmbH, 22045 Hamburg (DE)
(72) Inventor: ENDERS, Borg, Hamburg (DE); HARMSEN, Stefan, Warmenhuizen (NL); SOBAJIMA, Hideo, Tokyo (JP); HOGERVORST, Jordi, Hamburg (DE)
(74) Representative: Seemann & Partner Patentanwälte mbB

(57) **Abstract**

The present invention relates to a method and a system for processing fluorescent image guided surgical or diagnostics imagery. The method comprises capturing non-fluorescent images and fluorescent images of an operating field in which at least one fluorescent dye is present with a surgical or diagnostics imaging device, processing the fluorescent images with respect to brightness and coloring and generating composite images by overlaying the fluorescent images over the non-fluorescent images, wherein the processing of the fluorescent images involves inputting the fluorescent images into at least one artificial intelligence model trained on fluorescent still images and/or fluorescent video images to identify one or more types of structures by their fluorescent dye emission and performing color coding of the fluorescent images by coloring fluorescent parts of the fluorescent images with pre-determined false colors assigned to different types of structures according to the respective one or more identified types of structures.

## Description

The present disclosure relates to a method and a system for processing fluorescent image guided surgical or diagnostics imagery.

Medical applications of fluorescence imaging, a subtype of molecular imaging, are fluorescent image guided surgery and diagnostics, in which a medical imaging technique used to detect fluorescent substances with the purpose of guiding the surgical or diagnostic procedure and providing the operator with real time visualization of the operating field, both in open surgery and in endoscopic procedures, as well as in diagnostic procedures. Fluorescence dyes (fluorophores) commonly used for different applications in fluorescent image guided surgery and diagnostics include indocyanine green (ICG) and others in the optical and near infrared spectrum, but can also include dyes that fluoresce at other wavelengths, such as the ultraviolet or the far infrared spectrum or any wavelengths that can be processed by an image processor.

Fluorescence imaging is a form of molecular imaging, which generally encompasses imaging methods for visualizing and/or tracking of molecules having specific properties that are used for molecular imaging. Such molecules can be substances that are endogenous to the body, or dyes or contrast agents that are injected into the patient. MRI and CT, for example, therefore also fall under the term "molecular imaging". Fluorescence imaging as a variant of molecular imaging uses the property of certain molecules (fluorophores), which emit light of certain wavelengths when excited by light of certain excitation wavelengths.

For the purpose of fluorescence imaging, the system's imaging system, e.g., a camera head, typically includes sensors that are sensitive in the visible spectrum and in the near infrared spectrum, but may also cover other spectra, depending on the dye used. The system's illumination light source unit has a light source for white light to illuminate the operating area with white light as well as at least one excitation light source designed for exciting at least one fluorescent dye present in the operating area. The excitation light source may comprise a laser or a light emitting diode, the wavelength chosen to excite fluorescence in the dye being used. After being excited, the dyes shed the excitation energy by emitting light at slightly longer wavelengths than that of the excitation light. Other wavelengths may be used as excitation wavelengths depending on the type of dye used. This can include wavelengths that are further inside the visible spectrum or further outside the visible or infrared spectrum.

Based on the foregoing, the image processing system generates a composite image with an overlay of the fluorescent image over a non-fluorescent image for easy localization of the fluorescent areas within its surroundings and displays it to the surgeon on a screen. When overlaid over the white light images, the fluorescent images are usually converted into a false color image, for example in a light green color in the case of ICG as a dye, that ideally contrasts the red hues of the white light image, with the brightness indicating the intensity of the fluorescent emission.

Various dyes with different properties including different fluorescence excitation and emission spectra are available to highlight different structures during surgery, like the usage for lung or colon, or for highlighting tumors vs. structures like the Ureter. The dye information is shown on the screen as processed by the imaging sensor with only a small amount of post processing, e.g., by raising low intensity signals to a higher intensity for better visibility. Accordingly, the coloring shown on the monitors of imaging systems having image sensors with different sensibilities and excitation light sources with different excitation wavelengths may differ, further depending on the dye and its excitation wavelength.

Consequently, in order to correctly interpret the information displayed in the composite images, the surgeon needs to be familiar with the specific limitations and characteristics of the dyes and their coloring on the specific system. The surgeon needs multiple procedures to correctly interpret the information for even a single dye on top of the information, when and how best to apply dyes. This challenge is even further increased if more than one dye is used during surgery to highlight different structures, e.g. for example in colon surgery for ureter and tumor identification.

It is an object to provide improved methods and systems for processing fluorescent image guided surgical or diagnostics imagery that alleviate some or all of the aforementioned problems.

Such object can be solved by a method for processing fluorescent image guided surgical or diagnostics imagery, comprising capturing non-fluorescent images and fluorescent images of an operating field in which at least one fluorescent dye is present with a surgical or diagnostics imaging device, processing the fluorescent images with respect to brightness and coloring and generating composite images by overlaying the fluorescent images over the non-fluorescent images, wherein the processing of the fluorescent images involves inputting the fluorescent images into at least one artificial intelligence model trained on fluorescent still images and/or fluorescent video images to identify one or more types of structures by their fluorescent dye emission and performing color coding of the fluorescent images by coloring fluorescent parts of the fluorescent images with pre-determined false colors assigned to different types of structures according to the respective one or more identified types of structures

With this method, fluorescent images taken with surgical or diagnostics imaging devices such as video endoscopes, endoscopic systems having a camera head or exoscopes for observing open surgery are processed in real time for easy understanding by the surgeon, since different structures are colored differently and consistently with pre-determined colors. The automated identification of structures and their structured coloring helps making surgeries safer because the surgeon is presented with immediately recognizable and distinguishable visual information. This will reduce the learning curve for the surgeon for usage of new dyes and usages or purposes of dye use. The surgeon only has to familiarize himself or herself with the scheme of pre-determined colors, which may even already be known to him or her if the color scheme conforms to or is similar to a color scheme in a text book, thus further reducing the time spent learning the scheme. This also significantly reduces the risk of misinterpreting data, since the image generated by the artificial intelligence model will be more intuitive to the surgeon.

By training the at least one artificial intelligence model, which may be a convolutional neural net or any Al algorithm type able to process image data, on appropriately labelled training data, the at least one model is trained to identify structures labelled with fluorescent dyes in the body by the entirety of their appearance. This includes information of the fluorescence emission spectrum of the dyes used for the specific structures as well as their shape and other parameters that like structures have in common.

In an embodiment, the method comprises inputting the non-fluorescent images into the at least one artificial intelligence model such that each non-fluorescent image is coupled to a fluorescent image, the at least one artificial intelligence model having been trained for using the non-fluorescent images as additional input for identifying the one or more types of structures. Coupling the non-fluorescent images to the fluorescent images and using the non-fluorescent images as further input for the artificial intelligence model, further strengthens the reliability of the identification of different types of structures marked by the fluorescent dyes.

In embodiments, the method comprises several artificial intelligence models that have been trained on at least one of different dyes, different structures and different imaging systems, processing the fluorescent images and combining their outputs. This sort of specialization reduces the amount of training needed for each individual artificial intelligence model. One or more of the several, i.e., two or more, artificial intelligence models may be fed with non-fluorescent images as well as additional input data. Within the context of the present disclosure, one or several artificial intelligence models may be trained with either only one of or any combination of different dyes, different structures and different imaging systems.

According to a further embodiment, information about at least one of the fluorescent dye or dyes and the imaging system used in a fluorescent image guided surgical or diagnostic procedure are provided as input to the at least one artificial intelligence model. This additional information will aid in narrowing down the scope of different types of structures that can be highlighted in the fluorescent images of an ongoing fluorescent image guided surgical or diagnostic procedure.

In further embodiments, the method comprises generating a color legend of the colors used for the structures identified in the fluorescent images and one of integrating the legend in the composite images and displaying the legend separately from the composite images. Alternatively or in addition, the method may comprise generating text labeling of the structures identified in the fluorescent images and integrating the text labelings into the composite images. This may be implemented in a teaching or training mode to help surgeons to acquaint themselves with the structured context sensitive coloring.

According to another embodiment, the method comprises selecting the pre-determined colors from a set of several coloring schemes upon request by the surgeon. Such coloring schemes may be filled in individually by a surgeon as a personalized coloring scheme, or be pre-formatted so as to conform to known coloring schemes from known and standard anatomy textbooks, thus making the coloring immediately recognizable to any surgeon who has learned anatomy using such textbooks.

In another aspect of the invention, the object can be solved by a system for processing fluorescent image guided surgical or diagnostics imagery, comprising a surgical or diagnostics imaging device and a light source capable of generating white light illumination as well as fluorescent dye excitation illumination for at least one fluorescent dye, a computer system running at least one artificial intelligence model trained to identify one or more types of structures by their fluorescent dye emission in fluorescent images generated by the surgical or diagnostics imaging device and inputted into the at least one artificial intelligence model, and a composer designed to generate composite images by overlaying the fluorescent images processed by the at least one artificial intelligence model over the non-fluorescent images, the computer system being configured to color the fluorescent parts of the fluorescent images with pre-determined false colors according to the respective one or more identified types of structures, wherein different pre-determined colors are assigned to different types of structures. The system may comprise a screen, such as a main surgical monitor used for displaying surgical or diagnostics imagery during fluorescent image guided surgery.

The system and its components embody the same features, characteristics, concepts and advantages as the above-described method.

By way of non-limiting examples, the surgical or diagnostics imaging device may be a video endoscope or a video exoscope or a combination of one of a telescope for endoscopic procedures, an exoscope and an attachment lens releasably attached to a camera head. The term "endoscope" is to be broadly construed as comprising various kinds of specialized rigid or flexible endoscopic instruments with fixed or changeable straight or sideways directions of view, such as laparoscopes, coloscopes, telescopes, a.o., that may either have distally located image sensors or optical means such as relay lens systems or fiber optics for relaying incoming light to the proximal end of the instrument, with image sensors either being located in a handle portion of the instrument or in a separate camera head that can be affixed to the instrument. Likewise, the term "exoscope" may mean a shortened version of a rigid endoscope whose field of view and focal length are set for overviewing an operating field in open surgery or similar imaging devices having the same purpose.

The computer system may have a graphical processor unit (GPU) and a frame grabber running the at least one artificial intelligence model and to compose a non-fluorescent image with color coded fluorescent overlays. A GPU and frame grabber are suitable platforms for Al processing. However, any suitable hardware components such as CPUs, dedicated AI chips or the like may be used for the purpose.

In embodiments, the composer is implemented one of in the at least one artificial intelligence model and in a separate set of instructions receiving input about the identified types of structures from the one or more artificial intelligence models.

The computer system may be configured to input the non-fluorescent images into the at least one artificial intelligence model such that each non-fluorescent image is coupled to a fluorescent image, the at least one artificial intelligence model having been trained for using the non-fluorescent images as additional input for identifying the one or more types of structures.

In an embodiment, the computer system is configured to run several artificial intelligence models that are trained on at least one of several different dyes, several different structures and several different imaging systems. Some or all of these artificial intelligence models may be trained to receive and process non-fluorescent images as additional input.

In embodiments, the computer system is configured to provide information about at least one of the fluorescent dye or dyes and the imaging system used in a fluorescent image guided surgical or diagnostic procedure as input to the at least one artificial intelligence model.

Furthermore, the computer system may be configured to generate a color legend of the colors used for the structures identified in the fluorescent images and one of to integrate the legend in the composite images and to display the legend separately from the composite images.

In particular for training purposes, but also for example as a display option to be chosen by an operator, the computer system may be configured to generate color coded text labeling of the structures identified in the fluorescent images and to integrate the text labeling into the composite images.

In embodiments, the computer system is configured to select the pre-determined colors from a set of several coloring schemes upon request by an operator.

In another aspect of the invention, the object can be solved by a non-volatile data storage medium containing instructions for a computer that are configured for causing the computer to perform the above described method. This aspect of the invention provides the computer system with the capability of executing the previously described functions and therefore embodies the same features, characteristics, concepts and advantages as the above-described method.

Further features will become evident from the description of embodiments, together with the claims and the appended drawings. Embodiments can fulfill individual features or a combination of several features.

The embodiments described below, without restricting the general intent of the invention, based on exemplary embodiments, wherein reference is made expressly to the drawings with regard to the disclosure of all details that are not explained in greater detail in the text.

In the drawings:
Fig. 1 illustrates an embodiment of a system for processing fluorescent image guided surgical or diagnostics imagery,
Fig. 2 illustrates an embodiment of an Al subsystem of the system of Fig. 1,
Figs. 3A to 3C illustrate an embodiment of multi-dye color coded fluorescence imagery, and
Fig. 4 illustrates another embodiment of color coded fluorescence imagery.

In the drawings, the same or similar types of elements or respectively corresponding parts are provided with the same reference numbers in order to prevent the item from needing to be reintroduced.

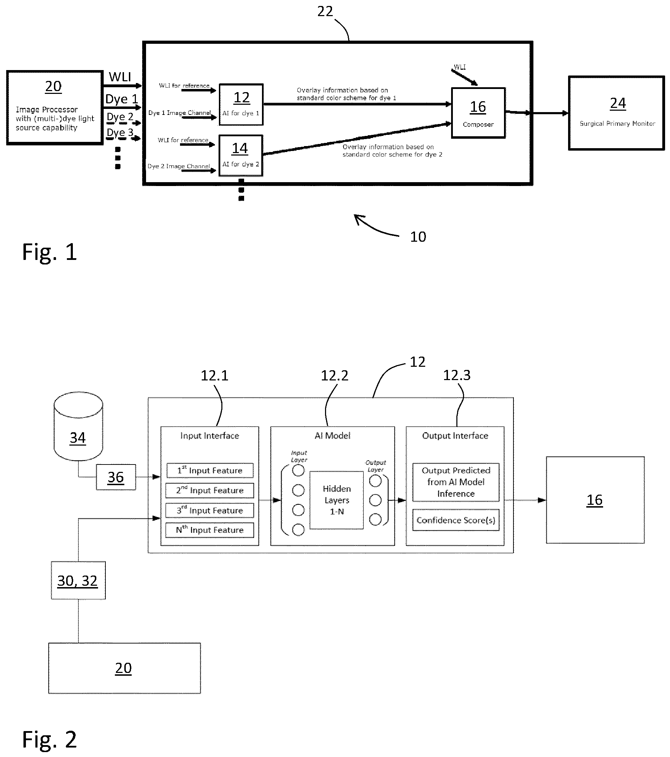

Fig. 1 illustrates an embodiment of a system 10 for processing fluorescent image guided surgical or diagnostics imagery. The system 10 comprises a surgical or diagnostics imaging device 20 having an image processor with single- or multi-dye light source capability that provides white light images (WLI) and fluorescent images stemming from one dye ("Dye 1") or, optionally, several dyes ("Dye 1", "Dye 2", "Dye 3", ...) to a computer system 22. The computer system 22 is a PC system with a graphical processing unit (GPU) and a frame grabber running artificial intelligence subsystems 12, 14 that separately process the fluorescent images with respect to different fluorescent dyes. Each artificial intelligence subsystem 12, 14 is provided with non-fluorescent images for reference and fluorescent images carrying the visual information provided by the fluorescent dyes. There may be more artificial intelligence subsystems if more than two different dyes are used. In contrast, instead of using multiple artificial intelligence subsystems 12, 14, ..., for the different dyes, a single artificial intelligence subsystem 12 may be trained to process the various dyes and imaging systems used during fluorescent image guided surgery.

The artificial image subsystems 12, 14 provide overlay information based on a standard color scheme for the different dyes and identified structures, which is provided to a composer 16, along with the non-fluorescent white light images. Composer 16 provides composite images in which the color coded fluorescent images are overlaid over the white light images. The composite images are provided to a surgical primary monitor 24 of system 20.

Instead of white light images as in the exemplary embodiment of Fig. 1, different illumination may be chosen for the non-fluorescent images, such as monochromatic or infrared lighting.

Fig. 2 illustrates an exemplary embodiment as a schematic diagram of an AI subsystem 12 of the system 10 of Fig. 1 that is configured to provide overlay information for color coded composite images with different colors indicating different structures that have been marked with fluorescent dyes and are identified in surgical images based on their appearance in fluorescent images 32. In various embodiments, the AI subsystem 12 includes an input interface 12.1 through which fluorescent images 32 produced by surgical or diagnostics imaging device 20 are provided as input features to an artificial intelligence (Al) model 12.2, which performs an inference operation in which the fluorescent images 32 are applied to the AI model 12.2 to generate the overlay information, and a output interface 12.3 through which overlay information is communicated to the composer 16 of Fig. 1.

The fluorescent images 32 may be accompanied by non-fluorescent images 30 and possibly other information which are specific to the ongoing surgical or diagnostic procedure as database input features 36 supplied by a database 34, such as the fluorescence features of the dyes, or information about the structure, the color scheme and/or the imaging system used.

In some embodiments, the input interface 12.1 may be a direct data link between the AI subsystem 12 and one or more medical devices 20 and databases 34 that generate at least some of the input features. For example, the input interface 12.1 may transmit fluorescent images 32 directly to the AI subsystem 12 during a surgical and/or diagnostic medical procedure. Additionally, or alternatively, the input interface 12 may be a classical user interface that facilitates interaction between a user and the computer system 22, which may facilitate the input of procedure related data by the operator that may be used as input to the AI model 12.2. For example, the input interface 12.1 may facilitate a user interface through which the user may manually enter the types of dyes used in the procedure. In any of these cases, the input interface 12.1 is configured to collect one or more of the following input features in association with a fluorescent image guided surgery or diagnostic procedure on or before a time at which the AI subsystem 12 is used to assess the fluorescent images.

Based on one or more of the above input features, the AI subsystem performs an inference operation using the AI model 12.2 to generate overlay information based on a standard color scheme for one or more dyes from the fluorescent images 32 and, if applicable, the non-fluorescent images 30 and other input data. For example, input interface 12.1 may deliver the fluorescent images 32 and, if applicable, correlated non-fluorescent images 30 and/or further input data such as the dye or dyes used into an input layer of the AI model 12.2, which propagates these input features through the AI model 12.2 to an output layer. The AI model 12.2 can provide the computer system 22 the ability to perform tasks, without explicitly being programmed, by making inferences based on patterns found in the analysis of data. AI model 12.2 explores the study and construction of algorithms (e.g., machine-learning algorithms) that may learn from existing data and make predictions about new data. Such algorithms operate by building an AI model from example training data in order to make datadriven predictions or decisions expressed as outputs or assessments.

There are two common modes for machine learning (ML): supervised ML and unsupervised ML. Supervised ML uses prior knowledge (e.g., examples that correlate inputs to outputs or outcomes) to learn the relationships between the inputs and the outputs. The goal of supervised ML is to learn a function that, given some training data, best approximates the relationship between the training inputs and outputs so that the ML model can implement the same relationships when given inputs to generate the corresponding outputs. Unsupervised ML is the training of an ML algorithm using information that is neither classified nor labeled, and allowing the algorithm to act on that information without guidance. Unsupervised ML is useful in exploratory analysis because it can automatically identify structure in data.

Common tasks for supervised ML are classification problems and regression problems. Classification problems, also referred to as categorization problems, aim at classifying items into one of several category values (for example, is this object an apple or an orange?). Regression algorithms aim at quantifying some items (for example, by providing a score to the value of some input). Some examples of commonly used supervised-ML algorithms are Logistic Regression (LR), Naive-Bayes, Random Forest (RF), neural networks (NN), deep neural networks (DNN), matrix factorization, and Support Vector Machines (SVM).

Some common tasks for unsupervised ML include clustering, representation learning, and density estimation. Some examples of commonly used unsupervised-ML algorithms are K-means clustering, principal component analysis, and autoencoders.

Another type of ML is federated learning (also known as collaborative learning) that trains an algorithm across multiple decentralized devices holding local data, without exchanging the data. This approach stands in contrast to traditional centralized machine-learning techniques where all the local datasets are uploaded to one server, as well as to more classical decentralized approaches which often assume that local data samples are identically distributed. Federated learning enables multiple actors to build a common, robust machine learning model without sharing data, thus allowing to address critical issues such as data privacy, data security, data access rights and access to heterogeneous data.

In some examples, the AI model 12.2 may be trained continuously or periodically prior to performance of the inference operation. Then, during the inference operation, the input features provided to the AI model 12.2 may be propagated from an input layer, through one or more hidden layers, and ultimately to an output layer that corresponds to the overlay information for the different dyes or structures. For example, the overlay information may include color information for each cluster of pixels showing fluorescence according to the structure identified to be associated with the specific type of fluorescence in the fluorescence images 32.

During and/or subsequent to the inference operation, the overlay information may cause the composer 16 to perform the color coded combination of fluorescent images 32 and non-fluorescent images 30.

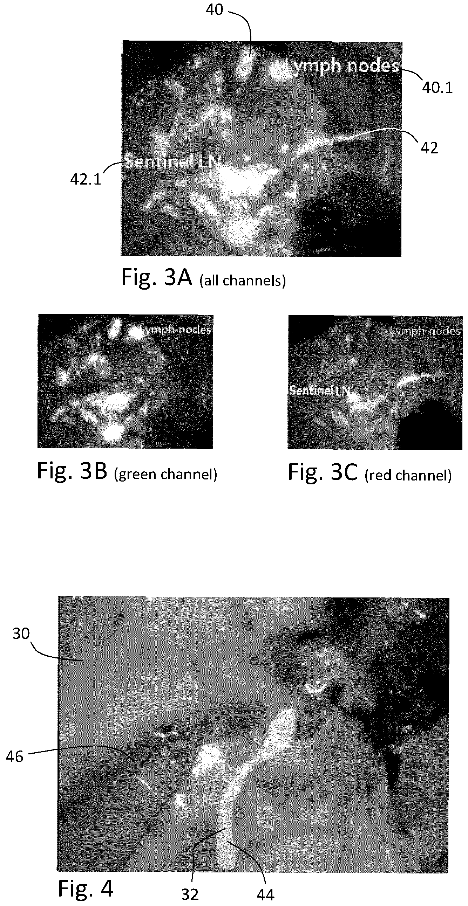

Figs. 3A to 3C illustrate an embodiment of multi-dye color coded fluorescence imagery that has been obtained using different fluorescent dyes for different structures. Fig. 3A illustrates in grey scales an overlay of fluorescent images obtained using two different dyes for two different structures over a non-fluorescent background image. The two different dyes attach to lymph nodes 40 on the one hand and to sentinel lymph nodes (sentinel LN) 42 on the other hand. The two different dyes fluoresce differently. In full color fluorescence imaging the lymph nodes 40 are represented in false green colors and the sentinel lymph nodes 42 in false red colors. Figs. 3B and 3C show the green and red channels of Fig. 3A, respectively, thereby displaying the lymph nodes 40 overlaid in green and the sentinel lymph nodes 42 overlaid in red separately from each other for the sake of clarity.

The composite image is further enhanced by legend texts 40.1, 42.1 for the lymph nodes 40 and the sentinel lymph node 42, respectively. The legend texts 40.1, 42.1 are displayed in the same green and red colors as the color used for the respective overlays.

Fig. 4 illustrates another embodiment of color coded fluorescence imagery. In this case a ureter 44 is marked with a specific fluorescent dye that appears in fluorescent image 32, which is in turn overlaid over white light image 30, which also shows a surgical instrument 46 next to the ureter 44. The fluorescent structure highlighting ureter 44 may be depicted in yellow, as is sometimes done in anatomy textbooks. The highlighting of the ureter 44 in yellow thereby helps the operator to immediately identify ureter 44 and avoid damaging it when operating in its vicinity.

While there has been shown and described what is considered to be embodiments of the invention, it will, of course, be understood that various modifications and changes in form or detail could readily be made without departing from the spirit of the invention. It is therefore intended that the invention be not limited to the exact forms described and illustrated, but should be constructed to cover all modifications that may fall within the scope of the appended claims.

### List of Reference Signs

- 10: System
- 12: AI subsystem
- 12.1: input interface
- 12.2: AI model
- 12.3: output interface
- 14: AI subsystem
- 16: composer
- 20: surgical or diagnostics imaging device
- 22: computer system
- 24: surgical primary monitor
- 30: WLI images
- 32: fluorescent images
- 34: fluorescence, structure and color scheme database
- 36: database input features
- 40: lymph nodes
- 40.1: lymph nodes legend text
- 42: sentinel lymph node
- 42.1: sentinel lymph node legend text
- 44: ureter
- 46: surgical instrument

## Claims

1. Method for processing fluorescent image guided surgical or diagnostics imagery, comprising
capturing non-fluorescent images and fluorescent images of an operating field in which at least one fluorescent dye is present with a surgical or diagnostics imaging device,
processing the fluorescent images with respect to brightness and coloring and
generating composite images by overlaying the fluorescent images over the non-fluorescent images,
wherein the processing of the fluorescent images involves inputting the fluorescent images into at least one artificial intelligence model trained on fluorescent still images and/or fluorescent video images to identify one or more types of structures by their fluorescent dye emission and
performing color coding of the fluorescent images by coloring fluorescent parts of the fluorescent images with pre-determined false colors assigned to different types of structures according to the respective one or more identified types of structures.

2. The method according to claim 1, comprising inputting the non-fluorescent images into the at least one artificial intelligence model such that each non-fluorescent image is coupled to a fluorescent image, the at least one artificial intelligence model having been trained for using the non-fluorescent images as additional input for identifying the one or more types of structures.

3. The method according to claim 1 or 2, comprising several artificial intelligence models that have been trained on at least one of several different dyes, several different structures and several different imaging systems.

4. The method according to one of claims 1 to 3, comprising providing information about at least one of the fluorescent dye or dyes and the imaging system used in a fluorescent image guided surgical or diagnostic procedure as input to the at least one artificial intelligence model.

5. The method according to one of claims 1 to 4, comprising generating a color legend of the colors used for the structures identified in the fluorescent images and one of integrating the legend in the composite images and displaying the legend separately from the composite images and/or generating color coded text labeling of the structures identified in the fluorescent images and integrating the text labeling into the composite images and/or selecting the pre-determined colors from a set of several coloring schemes upon request by an operator.

6. System for processing fluorescent image guided surgical or diagnostics imagery, comprising a surgical or diagnostics imaging device and a light source capable of generating white light illumination as well as fluorescent dye excitation illumination for at least one fluorescent dye, a computer system running at least one artificial intelligence model trained to identify one or more types of structures by their fluorescent dye emission in fluorescent images generated by the surgical or diagnostics imaging device and inputted into the at least one artificial intelligence model, and a composer designed to generate composite images by overlaying the fluorescent images processed by the at least one artificial intelligence model over the non-fluorescent images, the computer system being configured to color the fluorescent parts of the fluorescent images with pre-determined false colors according to the respective one or more identified types of structures, wherein different pre-determined colors are assigned to different types of structures.

7. The system according to claim 6, further comprising a screen.

8. The system according to claim 6 or 7, wherein the surgical or diagnostics imaging device is a video endoscope or a video exoscope or a combination of one of a telescope for endoscopic procedures, an exoscope and an attachment lens releasably attached to a camera head.

9. The system according to one of claims 6 to 8, wherein the composer is implemented one of in the at least one artificial intelligence model and in a separate set of instructions receiving input about the identified types of structures from the one or more artificial intelligence models.

10. The system according to one of claims 6 to 9, wherein the computer system is configured to input the non-fluorescent images into the at least one artificial intelligence model such that each non-fluorescent image is coupled to a fluorescent image, the at least one artificial intelligence model having been trained for using the non-fluorescent images as additional input for identifying the one or more types of structures.

11. The system according to one of claims 6 to 10, wherein the computer system is configured to run several artificial intelligence models that are trained on at least one of several different dyes, several different structures and several different imaging systems.

12. The system according to one of claims 6 to 11, wherein the computer system is configured to provide information about at least one of the fluorescent dye or dyes and the imaging system used in a fluorescent image guided surgical or diagnostic procedure as input to the at least one artificial intelligence model.

13. The system according to one of claims 6 to 12, wherein the computer system is configured to generate a color legend of the colors used for the structures identified in the fluorescent images and one of to integrate the legend in the composite images and to display the legend separately from the composite images and/or to generate color coded text labeling of the structures identified in the fluorescent images and to integrate the text labeling into the composite images and/or to select the pre-determined colors from a set of several coloring schemes upon request by an operator.

14. Non-volatile data storage medium containing instructions for a computer that are configured for causing the computer to perform the method according to one of claims 1 to 5.
